(19) Europäisches Patentamt / European Patent Office / Office européen des brevets

(11) **EP 3 888 669 A1**

(12) **EUROPEAN PATENT APPLICATION**
published in accordance with Art. 153(4) EPC

(43) Date of publication:
06.10.2021 Bulletin 2021/40

(21) Application number: 19890368.4

(22) Date of filing: 20.11.2019

(51) Int Cl.:
*A61K 36/488* (2006.01)   *A61K 36/346* (2006.01)
*A61P 19/08* (2006.01)   *A61P 5/24* (2006.01)
*A23L 33/105* (2016.01)

(86) International application number:
PCT/KR2019/015988

(87) International publication number:
WO 2020/111652 (04.06.2020 Gazette 2020/23)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 28.11.2018 KR 20180150175

(71) Applicant: Helixmith Co., Ltd.
Gangseo-gu
Seoul 07794 (KR)

(72) Inventors:
• MOON, Hobin
  Seoul 06270 (KR)
• LEE, Wonwoo
  Seoul 05587 (KR)
• LEE, Doo Suk
  Anyang-si Gyeonggi-do 14069 (KR)
• SON, Miwon
  Yongin-si Gyeonggi-do 16822 (KR)

(74) Representative: J A Kemp LLP
14 South Square
Gray's Inn
London WC1R 5JJ (GB)

(54) **COMPOSITION FOR PREVENTING OR TREATING METABOLIC BONE DISEASES OR MENOPAUSAL SYMPTOMS**

(57) The present invention relates to a composition for preventing or alleviating metabolic bone diseases or menopausal symptoms, comprising a complex extract of Pueraria Radix and Platycodon grandiflorum as an active ingredient. When the present invention is used, metabolic bone disease or menopausal symptoms can be effectively prevented or alleviated without side effects.

FIG. 1

EP 3 888 669 A1

**Description**

Technical Field

The present disclosure was made under Task No. HI16C0275 with the support of the Korean Ministry of Health and Welfare. The research management institution for the above project is the Korea Health Industry Development Institute, the research business name is "Guideline Center for Korean Medicine (Sharing Proven Health Technology and Medicine of Korean Medicine)", and the research project name is "Optimization Research through Quality Index Establishment and Validation of Saegmaeksangagambang for Developing Korean Medicine for Treating Menopausal Syndrome". The host institute is ViroMed CO., LTD. and the research period is from July 02, 2018 to November 30, 2018.

This application claims priority to and the benefit of Korean Patent Application No. 10-2018-0150175 filed in the Korean Intellectual Property Office on 28 November 2018, the disclosure of which is incorporated herein by reference.

The present disclosure relates to a composition comprising a complex extract of *Pueraria lobata* and *Platycodon grandiflorum* as an active ingredient for preventing or treating metabolic bone diseases or menopausal symptoms.

Background Art

The onset of metabolic bone diseases is accounted for by an imbalance in activity between osteoblasts and osteoclasts which are responsible for the formation and removal of bone tissues in the body, respectively. An osteoclast is a large multinuclear cell destroying or absorbing unnecessary osseous tissues during the metabolism of the bone. Mature osteoclasts are multinucleated, originating from a stem cell. After differentiation from mesenchymal hepatocytes, osteoblasts survive for about 34 months and form new bones at the site where activated osteoclasts break up old bones. A large number of osteoblasts form of an osteoid matrix which is gradually mineralized to complete osteogenesis. Thereafter, about 70% of osteoblasts die while some of osteoblasts differentiate into osteocytes and bone lining cells and survive. Since the amount of bones is maintained by balanced activities between osteoblasts and osteoclasts, it is important to develop a therapeutic agent targeting molecules which exerts significant influence on osteoclastic activity. That is, given an increased activity, osteoclasts responsible mainly for bone resorption accelerate the degradation of bones, causing osteoporosis characteristics of bone thinning and bone fracture. Therefore, studies have been focused on proteins regulatory of osteoclastic activity as targets for the therapy of bone diseases.

For example, osteopenia refers to a pre-stage of osteoporosis and the excessive resorption and formation of osteoclasts are known as a cause therefor. Bone atrophy in rheumatism is associated with excessive osteoclast resorption, as well. Fibrous dysplasia is results from vigorous osteoclastic activity. The suppression of osteoclast functions is used for therapy of Paget's disease and hypercalcemia. The suppression of excessive osteoclast formation and/or activation leads to the inhibition of neoplastic bone destruction. The increase in osteoclast resorption or differentiation causes osteolysis and osteoarthritis.

It is known that cancer cells having invaded into a bone proliferate in a microenvironment around the bone and stimulate osteoblast or osteoclast activities to determine whether to proceed to osteolytic bone metastasis or osteoblastic bone metastasis. Cancer cells having circulated along blood vessels settle to a bone and secrete osteolytic factors such as parathyroid hormone related protein (PTHrP), interleukin (IL)-1, IL-6, IL-8, and IL-11. The secreted factors induce osteoblasts to decrease in the expression of osteoprotegerin (OPG) and increase in the expression of a receptor activator of NF-kB ligand (RANKL). The increased RANKL binds to RANK of an osteoclast progenitor and matures a large number of osteoclast progenitors, resulting in bone destruction through an excessive bone resorption.

In addition, female menopause (climacteric), a type of endocrine syndrome, refers to a transitional period due to the decrease or loss of physiological and sexual function in women as the female hormone estrogen decreases due to the overall and gradual aging of the ovaries. Although not in the climacteric state, patients who have become deficient in estrogen due to other causes such as ovariectomy and hypo-ovarianism suffer from the same symptoms. Such menopausal symptoms include hot flashes, tachycardia, perspiration, or headaches, which are due to vascular changes, and muscle pain, arthralgia, and back pain, which are due to musculoskeletal changes. In addition, menopausal symptoms include urogenital change-related symptoms such as oliguria and incontinence and cranial nerve system change-related symptoms such as hypomnesia, depression, concentration decrease, and dizziness. Furthermore, menopausal women may suffer from amblyopia and skin and hair changes and even hormonal change-caused diseases such as menopausal osteoporosis and cardiovascular diseases, which may be fatal.

In order to improve physical and mental health and life quality of middle-aged women, there has been a need for the development of a therapeutic agent for alleviating menopausal symptoms. A hormone replacement therapy and medicines such as non-steroidal drugs have been developed to alleviate such menopausal symptoms. However, most of those medicines are known to have side effects such as headaches and weight gain. Particularly, estrogen replacement therapy, which is accounted for by artificial injection of a hormone, is known to increasing the risk of onset of metrorrhagia, stroke, heart failure, breast cancer, and uterine cancer as well as a rejection response.

Due to the aforementioned demerits, much attention has been paid to a natural estrogen consumption in a food or additive form in place of an estrogen therapy, and development of a novel menopausal medicine which is excellent in terms of alleviation of the menopausal symptoms without side effects is demanded.

The present inventors made intensive research efforts to search for a material which can prevent or alleviate metabolic bone diseases or menopausal symptoms and thus completed the present disclosure.

Detailed Description of the Invention

Technical Problem

Leading to the present disclosure, intensive and thorough research, conducted by the present inventors, into a natural substance for preventing, alleviating, and treating metabolic bone diseases or menopausal symptoms at excellent efficacy without side effects, resulted in the finding that a composition comprising a complex extract of *Pueraria lobata* and *platycodon grandiflorum* is highly effective for preventing, alleviating, or treating metabolic bone diseases or menopausal symptoms.

Therefore, an aspect of the present disclosure is to provide a pharmaceutical composition for preventing or treating metabolic bone diseases or menopausal symptoms.

Another aspect of the present disclosure is to provide a food composition for preventing or alleviating metabolic bone diseases or menopausal symptoms.

Still another aspect of the present disclosure is to provide a method for preventing, alleviating, or treating metabolic bone diseases or menopausal symptoms.

Technical Solution

An aspect of the present disclosure provides a pharmaceutical composition for preventing or treating metabolic bone diseases or menopausal symptoms, the composition including: (a) a complex extract of *Pueraria lobata* and *Platycodon grandiflorum* as an active ingredient; and (b) a pharmaceutically acceptable carrier thereof.

Leading to the present disclosure, intensive and thorough research, conducted by the present inventors, into a natural substance for preventing, alleviating, and treating metabolic bone diseases or menopausal symptoms at excellent efficacy without side effects, resulted in the finding that a composition comprising a complex extract of *Pueraria lobata* and *platycodon grandiflorum* is highly effective for preventing, alleviating, or treating metabolic bone diseases or menopausal symptoms.

The term *"Platycodon grandiflorum"*, as used herein, refers to a root of *Platycodon grandiflorum* A. DC., which is a plant of the family Campanulaceae, called *Kilkyoung* in Korea, *Jiegeng* in China, and *Kykyo* in Japan, and has been used as a traditional oriental medicine. *Platycodon grandiflorum* has been used as an expectorant, an antitussive, and a therapeutic for treatment of coughing and bronchitis.

The term *"Pueraria lobata"*, as used herein, refers to a tuber of kudzu (Pueraria thunbergiana BENTH), which is a perennial climbing plant of the family Leguminosae, contains substances such as puerarin, puerarin xylose, daidzein, sitosterol, and the like, and is known to exhibit hyperkinemic, contraparetic, and antipyretic activities.

The composition of the present disclosure includes a complex extract of *Pueraria lobata* and *Platycodon grandiflorum* as an active ingredient. The complex extract of *Pueraria lobata* and *Platycodon grandiflorum* may be prepared (i) using a single extraction process in which a mixture of *Pueraria lobata* and *Platycodon grandiflorum* is subjected to extraction with an extraction solvent or (ii) by preparing single component extracts of *Pueraria lobata* and *Platycodon grandiflorum* separately and then mixing them.

As used herein, the term "extract" commonly refers to a crude extract in the art as stated above, but is intended to encompass, in a broad sense, a fraction which is formed by additional fractionation of the extract. That is, the complex extract of *Pueraria lobata* and *Platycodon grandiflorum* may be obtained not only using the solvent but also by additionally performing a purifying process thereon. For example, the complex extract of *Pueraria lobata* and *Platycodon grandiflorum* of the present disclosure includes a fraction obtained by passing the extract through an ultrafiltration membrane with a predetermined molecular weight cut-off value, or a fraction obtained by additionally performing various purification methods such as various chromatographies (designed for separation according to size, charge, hydrophobicity, or hydrophilicity).

According to an embodiment, the extract of the present disclosure is a polar organic solvent extract. The "polar organic solvent", as used herein, includes (a) water, (b) an anhydrous or hydrated lower alcohol of 1-4 carbon atoms (e.g., methanol, ethanol, propanol, butanol, normal-propanol, iso-propanol, normal-butanol, etc.), and (c) acetic acid, or a mixture of the polar aprotic solvents.

In an embodiment, the polar organic solvent of the present disclosure is water, an anhydrous or hydrated lower alcohol of 1-4 carbon atoms, and acetic acid, or a mixture of two or more selected from the stated components.

In an embodiment of the present disclosure, the anhydrous or hydrated lower alcohol of 1-4 carbon atoms is at least one selected from the group consisting of methanol, ethanol, propanol, butanol, normal-propanol, iso-propanol, and normal-butanol.

The concentration of the organic solvent may be, but is not limited to, 1 to 100% (v/v), specifically 10 to 100% (v/v), 20 to 100% (v/v), 30 to 100% (v/v)), 40 to 100%(v/v), 50 to 100%(v/v), 60 to 100%(v/v), 70 to 100%(v/v), or 80 to 100%(v/v), and more specifically 25%(v/v), 50%(v/v), 75%(v/v), or 95%(v/v).

In order to prepare the extract according to an embodiment of the present disclosure, the amount of the solvent may be appropriately selected depending on an amount of medicinal herb components used therefor. Specifically, for example, the amount of the solvent may be, but is not limited to, a 1 to 20 volumes, more particularly, 2 to 20 volumes, even more particularly, 5 to 15 volumes, even far more particularly 7 to 12 volumes of the total weight of *Pueraria lobata* and *Platycodon grandiflorum* used to prepare the extract.

The extraction temperature for the extract of the present disclosure is not particularly limited and may be, for example, 0°C to 120°C, and, specifically, 15°C to 95°C.

In an embodiment of the present disclosure, the extraction temperature is preferably 80°C or higher for water used as an extraction solvent, and 15°C to 30°C for a lower alcohol of 1-4 carbon atoms used as an extraction solvent.

No particular limitations are imparted to the extraction time. For example, the extraction time may be 1 hour to 10 days, 1 hour to 120 hours, and specifically 1 hour to 72 hours, 1 hour to 48 hours, 1hour to 36 hours, 1 hour to 24 hours, 1 hour to 12 hours, 1 hour to 10 hours, or 1 hour to 6 hours, but is not limited thereto.

The extract used in the present disclosure can be obtained through hot water extraction, cold extraction, reflux cold extraction, ultrasonic extraction, or a conventional extraction method well known in the art. In an embodiment, the extract of the present disclosure can be obtained through cold extraction using a lower alcohol or through hot water extraction, and the extraction may be repeated for 1-10 rounds.

The complex extract of *Pueraria lobata* and *Platycodon grandiflorum* may be prepared into a powder form through an additional process such as a vacuum distillation and lyophilization, or spray drying.

In an embodiment of the present disclosure, active ingredients, *Pueraria lobata* and *Platycodon grandiflorum* are mixed at a weight ratio (w/w) of 1:20 to 20:1. In another embodiment, *Pueraria lobata* and *Platycodon grandiflorum* are mixing at weight ratio (w/w) of 1:15 to 15:1, 1:12 to 12:1, 1:10 to 10:1, 1:10 to 5:1, 1:10 to 3:1, 1:10 to 1:2, 1:10 to 1:1, 1:8 to 8:1, 1:8 to 5:1, 1:8 to 3:1, 1:8 to 1:2, 1:8 to 1:1, 1:6 to 6:1, 1:6 to 5:1, 1:6 to 3:1, 1:6 to 2:1, 1:6 to 1:1, 1:5 to 5:1, 1:5 to 3:1, 1:5 to 2:1, 1:5 to 1:1, 1:4 to 4:1, 1:4 to 3:1, 1:4 to 2:1, 1:4 to 1:1, 1:3 to 3:1, 1:3 to 2:1, 1:3 to 1:1, 1:2 to 2:1, 1:2 to 1:1, 1:1.5 to 1.5:1, or 1:1.

In an embodiment of the present disclosure, *Pueraria lobata* and *Platycodon grandiflorum* is mixed at a weight ratio of 1:1 to 1:8, more particularly 1:1, 1:2, 1:4, 1:8, 2:1, 4:1, or 8:1, and most particularly 1:1, but without limitations thereto.

The term "weight ratio (w/w)" as used in the context of mixing herein refers to a weight ratio of single ingredients before undergoing an extraction process. In the case of preparing the complex extract through a single extraction process in which a mixture of *Pueraria lobata* and *Platycodon grandiflorum* is extracted using an extraction solvent, for example, the mixing weight ratio refers to a weight ratio of respective single-components of *Pueraria lobata* and *Platycodon grandiflorum* included in the mixture. In the case in which the complex extract is prepared by separately extracting each of *Pueraria lobata* and *Platycodon grandiflorum* and mixing single extracts the mixing weight ratio refers to a weight ratio between "single component reference weights" calculated by a formula below:

[Formula]

Single component reference weight = weight of single component used for preparation of single extraction $\times$ (weight of single extract used for preparing final complex extract/weight of prepared single extract).

The composition of the present disclosure can be produced as a pharmaceutical composition.

The term "prevention" in the present disclosure includes all kinds of actions of inhibiting or delaying the onset of metabolic bone disease or menopausal symptoms by administration of a composition including the complex extract of *Pueraria lobata* and *Platycodon grandiflorum* according to the present disclosure as an active ingredient.

The term "treatment" in the present disclosure includes all kinds of actions to improve or positively change metabolic bone diseases or menopausal symptoms by administrating a composition including the complex extract of *Pueraria lobata* and *Platycodon grandiflorum* according to the present disclosure as an active ingredient.

The pharmaceutical composition of the present disclosure includes the complex extract of *Pueraria lobata* and *Platycodon grandiflorum* at a pharmaceutically effective amount. The "pharmaceutically effective amount" refers to an amount sufficient to exhibit the efficacy or activity of the complex extract of *Pueraria lobata* and Platycodon grandiflorum, but it is not particularly limited as long as it achieves the purpose of the present disclosure.

The pharmaceutical composition of the present disclosure includes a pharmaceutically acceptable carrier. The pharmaceutically acceptable carrier included in the pharmaceutical composition of the present disclosure pertains to a commonly used material for formulation and includes lactose, dextrose, sucrose, sorbitol, mannitol, starch, acacia rubber, calcium phosphate, alginate, gelatin, calcium silicate, microcrystal cellulose, polyvinyl pyrrolidone, water, syrup, and mineral oil, but is not limited thereto. The pharmaceutical composition of the present disclosure may additionally include lubricants, humectants, sweetening agents, fragrances, emulsifiers, suspensions, preservatives, etc. Suitable and pharmaceutically acceptable carrier and agents are described in detail in Remington's Pharmaceutical Sciences (19th ed., 1995).

The suitable level of an effective amount of the pharmaceutical composition of the present disclosure can be determined variously depending on factors such as formulation types, an administration method, an age and body weight of a patient, a gender of a patient, severity of patient's disease, diet, time of administration, route of administration, discharge rate, and responsive sensitivity. The general dosage of the pharmaceutical composition of the present disclosure is within a range of 0.001-1000 mg/kg on an adult basis. In addition, the dosage for a human body can be calculated on the basis of animal experiments.

The pharmaceutical composition of the present disclosure may be formulated into a unit dosage form or produced to be incorporated into a multi-dosage container by using pharmaceutically acceptable carriers and/or excipients according to methods which can be easily implemented by a person of ordinary knowledge in the art to which the present disclosure pertains. In this case, the formulation may be a form of solutions in an oil or aqueous medium, suspensions, or syrups or emulsions, or a form of extracts, powders, granules, tablets, or capsules, and may further include dispersants or stabilizers.

The "metabolic bone diseases" in the present disclosure refer to bone-related diseases triggered by the imbalance between osteoblasts and osteoclasts. Specific examples thereof include pathological bone diseases which accelerate bone destruction, such as osteoporosis, arthritis, periodontal disease, fractures, or Paget's disease which are caused by excessive bone resorption of osteoclasts, but is not limited thereto.

The "osteoporosis" refers to a state in which the amount of bone is reduced and the strength of bones is weaken due to a qualitative change thereof, leading to a high occurrence possibility of fractures. Bones protect various organs in the body, serve as a storage unit for materials required in the body, such as calcium, and maintain constancy by the balance of bone-degrading osteoclasts and bone-forming osteoblasts each present in osseous tissues. Osteoporosis may occur and proceed when the activity balance between the two cells is broken and excessive bone destruction by osteoclasts occurs. RANKL (receptor activator of nuclear factor kappa-B ligand) is a key element of osteoporosis and is coupled to a receptor thereof to activate various transcription factors, thereby promoting differentiation of osteoclasts.

"Arthritis" refers to a joint disease accompanied by inflammation on at least one joint area. A general form of arthritis is osteoarthritis in which cartilage protecting a joint is gradually damaged or bones and ligaments constituting a joint are damaged due to degenerative changes, causing inflammation and pain. Arthritis, with regard to the purpose of the present disclosure, is a disease accompanied by bone loss in a joint area and may include osteoarthritis or rheumatoid arthritis, but is not limited thereto.

"Periodontal disease" refers to the inflammatory state of supportive tissue caused by bacteria, and can be separated into gingivitis and periodontitis. The cause of the disease is the formation of dental plaque by oral bacteria due to poor oral hygiene. Dental plaque refers to a mass of bacteria that grows after bacteria adhere to a tooth surface by using a sticky substance in the saliva as an adhesive. If left untreated, the plaque becomes inflamed and sometimes causes bleeding from the gums and bad breath. These symptoms are called gingivitis. As the gingivitis progresses further, the gap between the teeth and the gums becomes deeper, and a paradental cyst develops, and the periodontitis occurs because the bacteria causing the periodontal disease multiply. As periodontitis progresses, even weak stimuli such as brushing may cause bleeding and swelling of the gums, often turning into acute inflammation, causing pain. This inflammation lowers the function of making bones, the bone absorbing effect is increased, the alveolar bone becomes lower, and the alveolar bone is destroyed and eventually teeth are lost.

"Fractures" refer a fracturing of the bone in a state in which the continuity of the bone, the end plate, or the joint surface is abnormally broken. The causes of fractures include traumas such as traffic accidents, safety accidents caused by industrial accident, bone changes caused by diseases such as osteoporosis, bone cancer, metabolic disorders, and repetitive bone stress caused by sports or loads. In addition, a fracture status is, on the basis of a fracture line (line along bone tip generated by bone cutting), classified into crack fracture, greenstick fracture, transverse fracture, filamentous fracture, spiral fracture, segmental fracture, comminuted fracture, avulsion fracture, compression fracture, depression fracture, etc.

"Paget's disease" refers to a localized bone disease in which bone remodeling is excessively aggravated and the skeletal system of a wide area is invaded. The pathologic mechanism of Paget's disease is believed to be a combination of an excessive increase in bone resorption by osteoclasts with the ability to cleanse the bone and an increase in new bone formation by osteoblasts with bone-forming functions as a compensation thereto, and newly formed bones in the bone in Paget's disease are structurally disordered and are known to be very vulnerable to bone deformation and fracture.

"Menopausal symptoms" of the present disclosure include symptoms caused by vascular changes, such as hot flashes, tachycardia, perspiration, or headaches, and symptoms caused by musculoskeletal changes, such as muscle pain, arthralgia, and back pain. The menopausal symptoms also include symptoms caused by urogenital changes, such as oliguria or incontinence, and symptoms caused by changes in cranial nerve system, such as hypomnesia, depression, concentration decrease, and dizziness. Furthermore, symptoms such as amblyopia and skin and hair change may occur and diseases such as menopausal osteoporosis and cardiovascular diseases due to changes in hormones may occur to threaten women's health.

As identified as below, the complex extract of *Pueraria lobata* and *Platycodon grandiflorum* remarkably reduced the TRAP activity in an osteoclast differentiation experiment in which a macrophage cell line of a mouse was treated with RANKL, compared with a negative control group and a group treated with single extracts of *Pueraria lobata* and Platycodon grandiflorum, thereby identifying the treatment and alleviation efficacy for osteoporosis according to inhibition of osteoclast differentiation.

In addition, the complex extract of *Pueraria lobata* and *Platycodon grandiflorum* was identified to be remarkably excellent in terms of the vasorelaxation effects in a cardiovascular disease model using a thoracic aortic strip compared with a negative control group and a group treated with single extracts of *Pueraria lobata* and Platycodon grandiflorum respectively, thereby identifying the treatment and alleviation efficacy of menopausal cardiovascular diseases.

Furthermore, the complex extract of *Pueraria lobata* and *Platycodon grandiflorum* was identified to remarkably improve biochemical indicators for osteoporosis, such as ALP, Ca, MMP-9, and osteocalcin in an ovariectomized osteoporosis mouse model, compared with a negative control group and a group treated with single extracts of *Pueraria lobata* and *Platycodon grandiflorum,* thus identifying the treatment and alleviation efficacy of osteoporosis.

The complex extract of *Pueraria lobata* and *Platycodon grandiflorum* showed a synergistic effect for each indicator compared with the single extract of *Pueraria lobata* or *Platycodon grandiflorum.*

Another aspect of the present disclosure provides a food composition including the complex extract of *Pueraria lobata* and *Platycodon grandiflorum* as an active ingredient of the present disclosure for prevention or treatment of metabolic bone diseases or menopausal symptoms.

The term "alleviation" in the present disclosure includes all kinds of actions to at least reduce the level of pathogenesis of metabolic bone diseases or menopausal symptoms by administration of a composition including the complex extract of *Pueraria lobata* and *Platycodon grandiflorum* according to the present disclosure as an active ingredient.

When the composition of the present disclosure is prepared into a food composition, the food composition contains ingredients commonly added for food manufacturing and examples of the ingredients include proteins, carbohydrates, fats, nutrients, seasoning agents, and flavoring agents. Examples of the aforementioned carbohydrates include regular sugars, e.g., monosaccharides such as glucose, fructose, and so on; disaccharides such as maltose, sucrose, oligosaccharide, and so on; and polysaccharides such as dextrin, cyclodextrin, and so on; and sugar alcohol such as xylitol, sorbitol, erythritol, and so on. The flavoring agents may be a natural flavoring agent and a synthetic flavoring agent. For example, when the food composition of the present disclosure is prepared as a beverage, citric acid, liquid fructose, sugar, glucose, acetic acid, malic acid, fruit juice, and the like may be further included in addition to the active ingredients of the present disclosure.

The food composition of the present disclosure may use the complex extract of *Pueraria lobata* and *Platycodon grandiflorum* as it is included in the pharmaceutical composition of an aspect of the present disclosure, and thus descriptions of overlapping contents therebetween will be omitted to avoid excessive complexities.

Still another aspect of the present disclosure provides a method for preventing, treating, and alleviating metabolic bone diseases or menopausal symptoms, the method including a step of administering to a subject a pharmaceutical composition or food composition comprising the complex extract of *Pueraria lobata* and *Platycodon grandiflorum* described in the present disclosure as an active ingredient.

The definition for metabolic bone diseases or menopausal symptoms which are the target diseases of the treatment or alleviation method of the present disclosure is the same as the definition with respect to the target disease of the pharmaceutical or food compositions.

As used herein, the term "administration" or "administer" refers to direct administration of a therapeutically or alleviatively effective amount of a composition of the present disclosure to a subject (an individual) suffering from the target disease so that the same amount is formed in the body of the subject.

The "therapeutically effective amount" of a composition means a content of the composition that is sufficient to provide a therapeutic or prophylactic effect to a subject to which the composition is to be administered, and includes a "prophylactically effective amount". Also, as used herein, the term "subject" includes mammals such as a human, mouse, rat, guinea pig, dog, cat, horse, cow, pig, monkey, chimpanzee, baboon or rhesus monkey. Most specifically, the subject of the present disclosure is a human.

The method for preventing, treating, and alleviating metabolic bone diseases or menopausal symptoms includes a step of administering the pharmaceutical or food composition according to an aspect of the present disclosure, and thus descriptions of overlapping contents therebetween will be omitted to avoid excessive complexities.

Advantageous Effects

The features and advantages of the present disclosure are as follows:

(a) The present disclosure provides a pharmaceutical composition for preventing or treating metabolic bone diseases or menopausal symptoms.
(b) The present disclosure provides a food composition for preventing or alleviating metabolic bone diseases or menopausal symptoms.
(c) The present disclosure provides a method for preventing, alleviating, or treating metabolic bone diseases or menopausal symptoms.
(d) The use of the pharmaceutical composition or the food composition according to the present disclosure can prevent, alleviate, and treat metabolic bone diseases or menopausal symptoms without side effects.

Brief Description of the Drawings

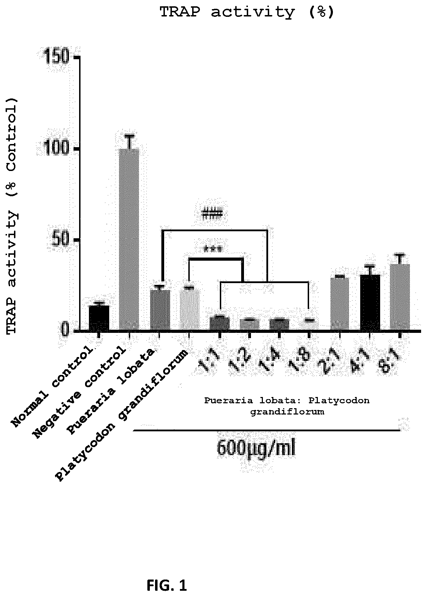

FIG. 1 shows a TRAP activity reduction effect of a complex extract of *Pueraria lobata* and *Platycodon grandiflorum* on differentiated osteoclasts according to Experimental Example 1. ### $P < 0.001$ vs. *Pueraria lobata* group; *** $P < 0.001$ vs. *Platycodon grandiflorum* group (one-way ANOVA, with Bonferroni's multiple comparison test).

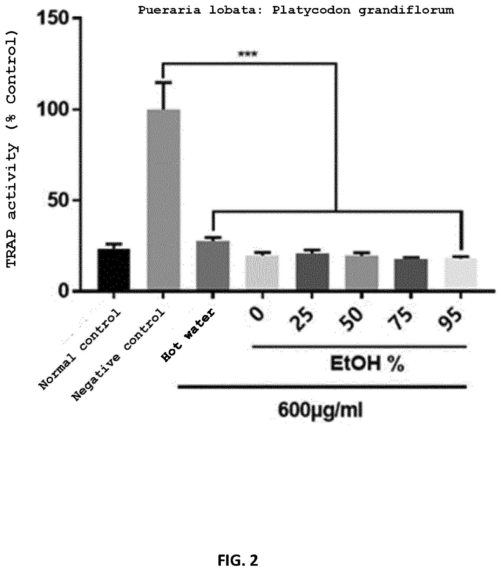

FIG. 2 shows a TRAP activity reduction effect of a complex extract for each of hot-water and ethanol concentrations on differentiated osteoclasts according to Experimental Example 1. *** $P < 0.001$ vs. negative control group (one-way ANOVA, with Bonferroni's multiple comparison test).

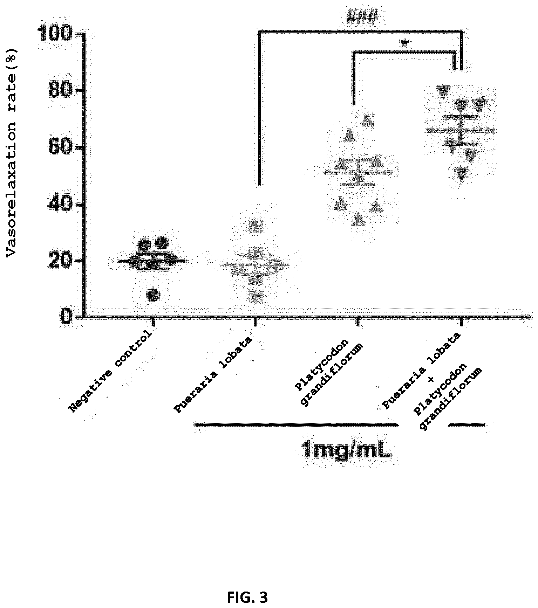

FIG. 3 shows a vasorelaxation effect of a complex extract of *Pueraria lobata* and *Platycodon grandiflorum* on a thoracic aorta section according to Experimental Example 2. ### $P < 0.001$ vs. *Pueraria lobata* group; * $P < 0.05$ vs. *Platycodon grandiflorum* group (one-way ANOVA, with Bonferroni's multiple comparison test).

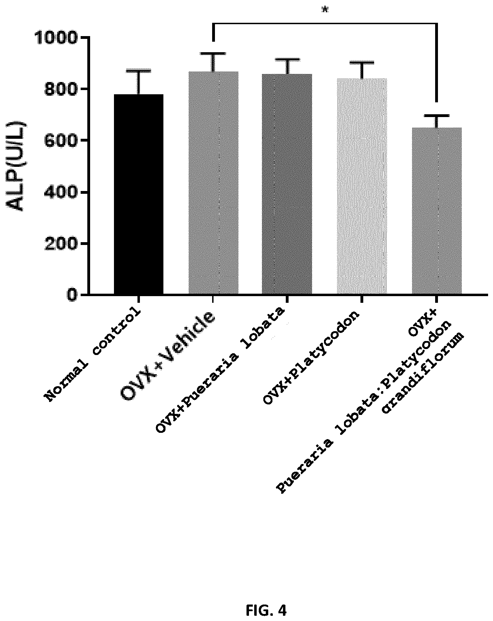

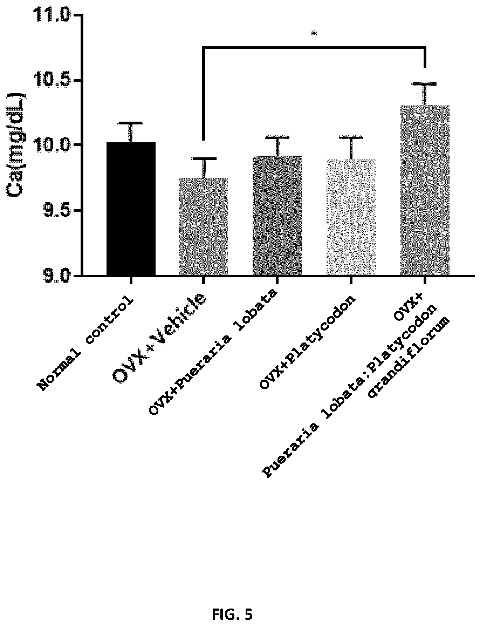

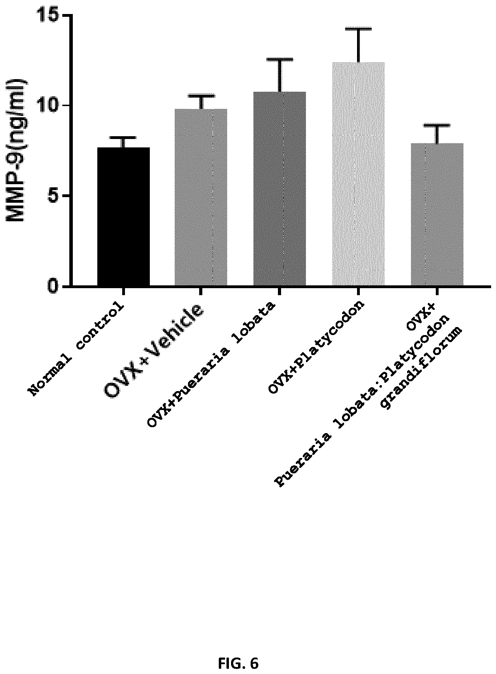

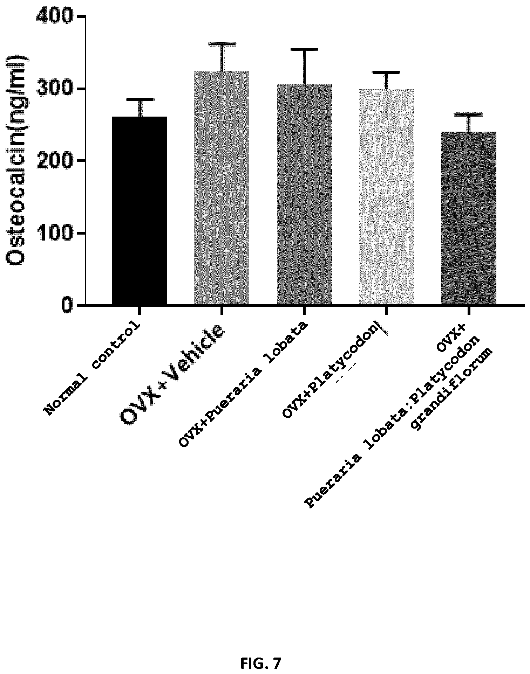

FIGS. 4 to 7 show an effect of a complex extract of *Pueraria lobata* and *Platycodon grandiflorum* on blood ALP (FIG. 4), Ca (FIG. 5), MMP-9 (FIG. 6), and osteocalcin (FIG. 7) concentrations compared with a ovariectomy group (OVX+Vehicle) according to Experimental Example 3. * $P < 0.05$ vs. negative control group (OVX+Vehicle) (one-way ANOVA, with Dunnett's multiple comparison test).

Mode for Carrying Out the Invention

Hereinafter, the present disclosure will be explained in detail with reference to examples. These examples are provided only for the purpose of illustrating the present disclosure in more detail, and therefore, according to the purpose of the present disclosure, it would be apparent to a person skilled in the art that these examples are not construed to limit the scope of the present disclosure.

**EXAMPLES**

**PREPARATION EXAMPLE 1: Preparation of Complex Extract of *Pueraria Lobata* and *Platycodon Grandiflorum***

Washed and dried *Pueraria lobata* and *Platycodon grandiflorum* were mixed at a weight ratio (w/w) of 1:1, 2:1, 4:1, 8:1, 1:2, 1:4, and 1:8 and added with 10 volumes 70% aqueous ethanol, followed by extraction at 20°C for 72 hours while stirring well. Thereafter, filtration was conducted before vacuum concentration at 45-50°C. Lyophilization afforded a complex extract powder. The yield is given in Table 1 below.

TABLE 1

| classification | Type of medicinal herb | Yield (%) |
|---|---|---|
| Preparation Example 1-1 | *Pueraria lobata · Platycodon grandiflorum* (1:1) | 21.82 |
| Preparation Example 1-2 | *Pueraria lobata · Platycodon grandiflorum* (1:2) | 18.99 |
| Preparation Example 1-3 | *Pueraria lobata · Platycodon grandiflorum* (1:4) | 20.08 |
| Preparation Example 1-4 | *Pueraria lobata · Platycodon grandiflorum* (1:8) | 19.52 |
| Preparation Example 1-5 | *Pueraria lobata · Platycodon grandiflorum* (2:1) | 23.48 |

(continued)

| classification | Type of medicinal herb | Yield (%) |
|---|---|---|
| Preparation Example 1-6 | *Pueraria lobata · Platycodon grandiflorum* (4:1) | 22.12 |
| Preparation Example 1-7 | *Pueraria lobata · Platycodon grandiflorum* (8:1) | 22.53 |

## COMPARATIVE EXAMPLE 1: Preparation of Single Extract

Washed and dried *Pueraria lobata* and *Platycodon grandiflorum* were added with 10 volumes 70% ethanol aqueous solution to each medicinal herb weight, followed by extraction 20°C for 72 hours while stirring well. Thereafter, filtration was conducted before vacuum decompression concentration at 45-50°C. Lyophilization afforded a total of two kinds of single extract powders. The yield is given in Table 2 below.

TABLE 2

| Classification | Type of medicinal herb | Yield (%) |
|---|---|---|
| Comparative example 1-1 | *Pueraria lobata* | 21.79 |
| Comparative example 1-2 | *Platycodon grandiflorum* | 21.56 |

## PREPARATION EXAMPLE 2: Preparation of Complex Extract of *Pueraria Lobata* and *Platycodon Grandiflorum* Using Various Extract Solvents

Washed and dried *Pueraria lobata* and *Platycodon grandiflorum* were mixed at a weight ratio (w/w) of 1:1. For a hot water extract, the mixture was subjected to extraction under reflux at $90 \pm 5$°C for 3 hours. For an ethanol extract, the mixture was added with 10 volumes of 0, 25, 50, 75, 95% aqueous ethanol before extraction at a 20°C for 72 hours while stirring well. Then, concentration in a vacuum at 45-50°C was followed by lyophilization to give a complex extract powder. The yield is given in Table 3 below.

TABLE 3

| Classification | Type of medicinal herb | Extract solvent | Yield (%) |
|---|---|---|---|
| Preparation Example 1-8 | *Pueraria lobata Platycodon grandiflorum* (1:1) | Distilled water (hot water) | 28.31 |
| Preparation Example 1-9 | *Pueraria lobata Platycodon grandiflorum* (1:1) | 0% Ethanol (cold extraction) | 24.68 |
| Preparation Example 1-10 | *Pueraria lobata Platycodon grandiflorum* (1:1) | 25% Ethanol (cold extraction) | 27.72 |
| Preparation Example 1-11 | *Pueraria lobata Platycodon grandiflorum* (1:1) | 50% Ethanol (cold extraction) | 25.20 |
| Preparation Example 1-12 | *Pueraria lobata Platycodon grandiflorum* (1:1) | 75% Ethanol (cold extraction) | 15.41 |
| Preparation Example 1-13 | *Pueraria lobata Platycodon grandiflorum* (1:1) | 95% Ethanol (cold extraction) | 2.996 |

## EXPERIMENTAL EXAMPLE 1: Inhibitory effect of complex extract on RANKL (receptor activator of NF-kappa B ligand, Sigma-Aldrich, US)-induced osteoclast differentiation

TRAP (tartrate-resistant acid phosphatase) is an enzyme used as an indicator of the osteoclast differentiation process, and the reduction in TRAP activity accounts for the inhibition of osteoclast differentiation. In this experiment, osteoclasts which were derived and differentiated by RANKL (receptor activator of NF-kappa B ligand, Sigma-Aldrich, US) from the mouse macrophage cell line RAW264.7 were used to identify effects of the single and complex extracts on osteoclast differentiation through measurement of TRAP activity, and effects of complex extracts on osteoclast differentiation for each solvent.

1-1. Cell Line Culture

First, RAW264.7 cells were incubated using a phenol-red free α-MEM (GIBCO, USA) medium supplemented with 10% charcoal-stripped fetal bovine serum (GIBCO) in a 5% $CO_2$ incubator at 37°C. RAW264.7 cells were plated to a 96-well plate at a density of 1x10$^3$ cells per well, and stabilized for 24 hours. Thereafter, the cells were treated with 50 ng/ml of RANKL and 600 µg/ml of each of single and complex extracts of *Pueraria lobata* and *Platycodon grandiflorum* or 600 µg/ml of each of a hot water complex extract and ethanol complex extracts of *Pueraria lobata* and *Platycodon grandiflorum* for 72 hours before TRAP activity analysis.

1-2. TRAP Activity

In brief, the cells were incubated with 30 µl of TRAP activity assay solution (sodium acetate 600 mM, pH 5.5, L-ascorbic acid 17.6 mg/ml, sodium-tartrate dehydrate 9.2 mg/ml, 4-nitrophenylphosphate Na 3.6 mg/ml, Triton X-100 0.3%, EDTA 6 mM, NaCl 600 mM) for 30 minutes in 37°C incubator. After the reaction was terminated with 300 mM NaOH absorbance was read at 450 nm wavelength.

TRAP activity is expressed as a ratio relative to the RANKL-treated group (negative control).

In the experiment for comparison between the single extracts and the complex extracts, treatment of RAW264.7 with RANKL increased TRAP activity, compared the untreated group, which was reduced in all of the extract-treated groups, as shown in Table 4 and FIG. 1. Particularly, the complex extract-treated groups further decreased in TRAP activity compared to groups treated with single extracts of *Pueraria lobata* or *Platycodon grandiflorum,* demonstrating that the complex extracts highly effectively inhibit osteoclast differentiation, compared with the single extracts.

TABLE 4

| Experimental Group | *Pueraria lobata : Platycodon grandiflorum* ratio | TRAP activity (%) |
|---|---|---|
| Untreated | - | 14.24± 1.49 |
| RANKL-treated | - | 100± 7.03 |
| *Pueraria lobata* extract (comparative example 1-1) | - | 22.59± 2.21 |
| *Platycodon grandiflorum* extract (comparative example 1-2) | - | 22.98± 1.04 |
| Complex extract (preparation example 1-1) | 1:1 | 7.76± 0.27 |
| Complex extract (preparation example 1-2) | 1:2 | 6.21± 0.21 |
| Complex extract (preparation example 1-3) | 1:4 | 6.29± 0.07 |
| Complex extract (preparation example 1-4) | 1:8 | 6.02± 0.05 |
| Complex extract (preparation example 1- 5) | 2:1 | 29.57± 0.64 |
| Complex extract (preparation example 1-6) | 4:1 | 30.99± 4.75 |
| Complex extract (preparation example 1-7) | 8:1 | 37.16± 4.87 |

As shown in following Table 5 and FIG. 2, remarkable TRAP activity reduction was found in all experimental groups treated with the hot-water extract (preparation example 1-8), and the cold-ethanol extracts according to concentrations (preparation examples 1-9, 1-10, 1-11, 1-12, and 1-13).

TABLE 5

| Experimental Group | *Pueraria lobata* : *Platycodon grandiflorum* ratio | TRAP activity (%) |
|---|---|---|
| Untreated | - | 23.75± 2.42 |
| RANKL-treated | - | 100± 14.72 |
| Hot water extract (preparation example 1-8) | 1:1 | 27.76± 1.98 |

(continued)

| Experimental Group | *Pueraria lobata* : *Platycodon grandiflorum* ratio | TRAP activity (%) |
|---|---|---|
| 0% Cold ethanol complex extract (preparation example 1-9) | 1:1 | 19.74± 1.74 |
| 25% Cold ethanol complex extract (preparation example 1-10) | 1:1 | 20.89± 1.90 |
| 50% Cold ethanol complex extract (preparation example 1-11) | 1:1 | 19.52± 1.78 |
| 75% Cold ethanol complex extract (preparation example 1-12) | 1:1 | 18.04± 0.68 |
| 95% Cold ethanol complex extract (preparation example 1-13) | 1:1 | 18.18± 0.95 |

In addition, correction of the values in Tables 4 and 5 confirmed similar TRAP activity between Preparation Example 1-1 in Table 4 and Preparation Example 1-12 of Table 5.

**EXPERIMENTAL EXAMPLE 2: Vasorelaxation Efficacy of *Pueraria lobata* and *Platycodon grandiflorum* Complex Extract**

The estrogen deficiency is a cause of blood pressure elevation in menopausal women by inducing vasoconstriction. Cerebrovascular constriction induces cerebral apoplexy. Therefore, a vasorelaxation efficacy is used as an important evaluation indicator in finding candidate materials of prophylactic and therapeutic drugs for menopausal cardiovascular diseases. In order to identify vasorelaxation efficacy of the complex extracts of *Pueraria lobata* and *Platycodon grandiflorum* of Preparation Example 1, phenylephrine (PE)-precontracted rat thoracic aortic strips were assayed for relaxation response in an organ bath.

2-1. Preparation of Thoracic Aortic Strip

Thoracotomy was performed on the experimental animals male Sprague Dawley (SD) rats at 8 weeks of age that were anesthetized with ethylether inhalation. Immediately after being excised, thethoracic aorta was supplied with a gas of 95% of $O_2$ and 5% of $CO_2$ and cleaned of adherent tissues and fats in Krebs-Henseleit solution (K-H solution, composition, mM: NaCl, 118.0; KCl, 4.7; $MgSO_4$, 1.2; $KH_2PO_4$, 1.2; $CaCl_2$, 2.5; $NaHCO_3$, 25.0 ; and glucose, 11.1; pH 7.4) maintained at 37°C. Subsequently, the aorta was cut into strips in a ring form about 2 mm in length.

2-2. Isotonic Contraction

The prepared aortic strip was hooked at the opposite ends thereof by respective tungsten wires, after which the lower portion thereof was anchored to a hook installed on the bottom of a 10-mL organ bath while the upper portion thereof was connected to an isometric force transducer (AD Instrument Co., Australia) connected to a physiograph (AD Instrument Co., Australia) for the measurement of isotonic contraction changes, which was recorded using the PowerLab program (AD Instrument Co., Australia). The strip was stabilized for 15 minutes in the organ bath and then loaded with a passive tension of 1 g. An experiment was proceeded after re-stabilization for 1 hour. The Krebs-Henseleit solution in the organ bath was replaced with a fresh one every 20 minutes during stabilization.

In order to identify a vasorelaxation activity therein, the aortic strip was treated with 1 $\mu$M phenylephrine for 40 minutes in the organ bath to induce vasocontraction. The sufficiently contracted strip was treated with 1 mg/ml of each of the single and complex extracts of *Pueraria lobata* and *Platycodon grandiflorum* in Krebs-Henseleit solution in the organ bath to compare vasorelaxation activities. The negative control was treated with Krebs-Henseleit solution alone. The vasorelaxation rate (%) was calculated by the following equation.

```
Equation
```

$$vasorelaxation\ rate\ (\%) = \frac{(B - A) - (C - A)}{(B - A)} \times 100$$

A: strip contraction before PE-induced contraction
B: strip contraction after PE-induced contraction
C: strip contraction after K-H solution or extract treatment

As shown in Table 6 and FIG. 3, treatment of the aortic strips with phenylephrine induced vasorelaxation at a rate of about 19.92%. Compared with the negative control, the single extract of *Pueraria lobata* exhibited no effects on vasorelaxation whereas treatment with the *Platycodon grandiflorum* extract and the complex extract increased the vasorelaxation rate by about 31.35% and about 46.26%, respectively, thus demonstrating the complex extract according to the present disclosure was superior to the negative control and the single extracts in terms of vasorelaxation efficacy.

TABLE 6

| Experimental Group | Strip contraction value (g) | | | Vasorelaxatio n rate (%) |
|---|---|---|---|---|
| | Before PE-induced contraction | After PE-induced contraction | After K-H solution or extract treatment | |
| Negative control | 1.13±0.02 | 3.38±0.11 | 2.94±0.1272 | 19.92±2.70 |
| *Pueraria lobata* extract (comparative example 1-1) | 1.24±0.02 | 3.51±0.19 | 3.11±0.20 | 18.60±3.42 |
| *Platycodon grandiflorum* extract (comparative example 1-2) | 1.20±0.03 | 3.25±0.09 | 2.21±0.13 | 51.27±4.37 |
| Complex extract (preparation example 1-1) | 1.17±0.04 | 3.57±0.25 | 1.99±0.17 | 66.18±4.77 |

**EXPERIMENTAL EXAMPLE 3: Efficacy of Complex Extract of *Pueraria lobata* and *Platycodon grandiflorum* on Bone Loss Marker**

Estrogen deficiency, which is characteristically observed in menopausal women, induces differentiation of osteoclasts degrading bones, resulting in bone loss and osteoporosis. The ovariectomized (OVX) rat model is currently the best model to reflect estrogen deficiency-induced bone loss symptoms in menopausal women. An ovariectomized rat model was observed in have lowered blood concentrations of calcium (Ca) and inorganic phosphate, which are constituents of bones, and increased levels of MMP-9 (Matrix metallopeptidase-9), which is a main enzymes for degrading bones, ALP (alkaline phosphatase), which is a bone turnover marker, and osteocalcin. These markers are used as main indices for evaluating bone loss. In order to examine an alleviative effect of the complex extracts of *Pueraria lobata* and *Platycodon grandiflorum* of Preparation Example 1 on bone loss in terms of the markers, biochemical analysis was conducted on the blood of the ovariectomized female rat.

3-1. Construction of Ovariectomized (OVX) Rat

Female Sprague Dawley rats at 6 weeks of age (each weighing about 160 g ± 20 %) were purchased from Orient (OrientBio, Gapyeong-gun, Gyeonggi-do) and acclimated for 14 days before experiments. The animals were anesthetized with Zoletil 50 (VIRBAC, France) and xylazine (Rompun®, Bayer AG, Germany) for ovariectomy. After the animals were shaved at both the abdominal and dorsal sides and disinfected at sites for surgery, incisions were made in the muscle and peritoneum to expose the uterus and ovaries to identify ovary loci. After exposure of the fallopian tubes and ovarian arteriovenous veins, the ovaries were excised using a cautery, followed by closing the wounds. The test substances, including single and complex extracts of *Pueraria lobata* (Comparative Example 1-1), *Platycodon grandiflorum* (Comparative Example 1-2), and *Pueraria lobata* and *Platycodon grandiflorum* (Preparation Example 1-1) were orally administrated once a day at a daily dose of 200 mg/kg for 7 days/week, 12 weeks. The normal group and the negative control were orally administered distilled water.

3-2. Blood Biochemical Test

After 12 weeks of administration, a serum separated from blood collected from the posterior vena cava was analyzed for alkaline phosphatase (ALP) and calcium, using a blood biochemical analyzer (7180 Hitachi, Japan) and an automatic electrolyte analyzer. In addition, the osteolysis markers MMP (Matrix metallopeptidase)-9 and Osteocalcin were measured by ELISA.

As shown in Table 7 and FIGS. 4 to 7, ovariectomy resulted in increasing blood levels of the osteoblast markers ALP and osteocalcin, which increase upon bone loss, and MMP-9, which is directly involved in the breakdown of bones, and decreasing blood levels of Ca, which is an inorganic constituent of bones. When the test substances were administered, there were no significant changes of ALP and Ca levels in the single extract-administered groups, but the markers were changed in a desired direction the complex extract-administered group with significance. In addition, the complex extract-administered group showed decreased blood levels of MMP-9 and osteocalcin markers. From the above results, it was confirmed that the complex extract of the present disclosure has excellent efficacy in improving indicators related to bone loss compared to the negative control and single extracts.

TABLE 7

| Experimental Group | ovariectomy (OVX) | Experimental material | ALP (U/L) | Ca (mg/dL) | MMP-9 (ng/mL) | Osteocalcin (ng/mL) |
|---|---|---|---|---|---|---|
| Normal | X | Distilled water | 782.01 ± 89.86 | 10.02 ± 0.15 | 7.65 ± 0.61 | 260.85 ± 23.94 |
| Negative control | O | Distilled water | 825.61± 76.23 | 9.74 ± 0.16 | 9.81 ± 0.73 | 332.96 ± 37.91 |
| *Pueraria lobata* extract (comparative example 1-1) | O | Comparative example 1-1 | 860.26 ± 49.10 | 9.93 ± 0.12 | 10.76 ± 1.79 | 305.26 ± 49.03 |
| *Platycodon grandiflorum* extract (comparative example 1-2) | O | Comparative example 1-2 | 841.26 ± 56.21 | 9.89 ± 0.14 | 12.38 ± 1.86 | 299.93 ± 22.68 |
| Complex extract (preparation example 1-1) | O | Preparation example 1-1 | 649.19 ± 43.50 | 10.30 ± 0.14 | 7.91 ± 1.00 | 239.89 ± 24.53 |

**Claims**

1.    A pharmaceutical composition for preventing or treating a metabolic bone disease or a menopausal symptom, the composition comprising:

(a) a complex extract of *Pueraria lobata* and *Platycodon grandiflorum* as an active ingredient; and
(b) a pharmaceutically acceptable carrier thereof.

2.    The composition of claim 1, wherein a weight ratio (w/w) of *Pueraria lobata* to *Platycodon grandiflorum* in the complex extract is 1:20 to 20:1.

3.    The composition of claim 1, wherein the extract is a polar organic solvent extract.

4.    The composition of claim 3, wherein the polar organic solvent is water, an anhydrous or hydrated lower alcohol of 1-4 carbon atoms, acetic acid, or a mixture thereof.

5.    A food composition for preventing or alleviating a metabolic bone disease or a menopausal symptom, the composition comprising a complex extract of *Pueraria lobata* and *Platycodon grandiflorum* as an active ingredient.

6.    The food composition of claim 5,
wherein a weight ratio (w/w) of *Pueraria lobata* to *Platycodon grandiflorum* in the complex extract is 1:20 to 20:1.

7. The food composition of claim 5, wherein the extract is a polar organic solvent extract.

8. The food composition of claim 7, wherein the polar organic solvent is water, an anhydrous or hydrated lower alcohol of 1-4 carbon atoms, acetic acid, or a mixture thereof.

FIG. 1

FIG. 2

FIG. 3

FIG. 4

**FIG. 5**

**FIG. 6**

**FIG. 7**

## INTERNATIONAL SEARCH REPORT

| International application No. |
|---|
| PCT/KR2019/015988 |

**A.  CLASSIFICATION OF SUBJECT MATTER**

*A61K 36/488(2006.01)i, A61K 36/346(2006.01)i, A61P 19/08(2006.01)i, A61P 5/24(2006.01)i, A23L 33/105(2016.01)i*

According to International Patent Classification (IPC) or to both national classification and IPC

**B.  FIELDS SEARCHED**

Minimum documentation searched (classification system followed by classification symbols)
A61K 36/488; A23K 10/12; A23K 10/30; A61K 31/704; A61K 35/78; A61K 36/27; A61K 36/346; A61P 19/10; A61P 3/04; A61P 19/08; A61P 5/24; A23L 33/105

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: Pueraria lobata, Platycodon grandiflorum, metabolic bone disease, climacteric

**C.  DOCUMENTS CONSIDERED TO BE RELEVANT**

| Category* | Citation of document, with indication, where appropriate, of the relevant passages | Relevant to claim No. |
|---|---|---|
| X | KR 10-2015-0045036 A (KOREA UNIVERSITY RESEARCH AND BUSINESS FOUNDATION) 28 April 2015 See paragraphs [0008], [0025]-[0026], [0028], [0046]-[0047]; claims 1, 4, 7, 10; and table 1. | 1-8 |
| X | KR 10-1776277 B1 (KBIOS CO., LTD.) 11 September 2017 See paragraphs [0015]-[0017], [0116], [0118]; and claims 1, 7. | 1-8 |
| A | 충남대학교 산학협력단, 길경과 갈근을 이용한 골 형성 촉진 및 골다공증 예방 기능성 식품 소재 개발, 보건의료기술진흥사업 최종보고서 (THE INDUSTRY & ACADEMIC COOPERATION IN CHUNGNAM NATIONAL UNIVERSITY. Development of Functional Food Material Containing Ossification Stimulation Effects by Platycodon Grandiflorum and Pueraria Radix. Health & Medical Technology R&D Program Final Report). 2011 See the entire document. | 1-8 |
| A | KR 10-2010-0069202 A (SEOUL NATIONAL UNIVERSITY R&DB FOUNDATION et al.) 24 June 2010 See the entire document. | 1-8 |
| A | JP 2005-325107 A (TAISHO PHARMACEUT CO., LTD.) 24 November 2005 See the entire document. | 1-8 |

☐  Further documents are listed in the continuation of Box C.      ☒  See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 17 MARCH 2020 (17.03.2020) | **17 MARCH 2020 (17.03.2020)** |

| Name and mailing address of the ISA/KR | Authorized officer |
|---|---|
| Korean Intellectual Property Office Government Complex Daejeon Building 4, 189, Cheongsa-ro, Seo-gu, Daejeon, 35208, Republic of Korea Facsimile No. +82-42-481-8578 | Telephone No. |

Form PCT/ISA/210 (second sheet) (January 2015)

INTERNATIONAL SEARCH REPORT
Information on patent family members

International application No.

**PCT/KR2019/015988**

| Patent document cited in search report | Publication date | Patent family member | Publication date |
|---|---|---|---|
| KR 10-2015-0045036 A | 28/04/2015 | KR 10-1544532 B1 | 17/08/2015 |
| KR 10-1776277 B1 | 11/09/2017 | None | |
| KR 10-2010-0069202 A | 24/06/2010 | KR 10-1056362 B1 | 11/08/2011 |
| JP 2005-325107 A | 24/11/2005 | JP 4892856 B2 | 07/03/2012 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020180150175

### Non-patent literature cited in the description

- Optimization Research through Quality Index Establishment and Validation of Saegmaeksangagambang for Developing Korean Medicine for Treating Menopausal Syndrome. institute is ViroMed CO., LTD, 02 July 2018

- Remington's Pharmaceutical Sciences. 1995